# EUROPÄISCHE PATENTSCHRIFT

(11) **EP 2 106 705 B1**
(45) Veröffentlichungstag und Bekanntmachung des Hinweises auf die Patenterteilung: **04.01.2012**
(21) Anmeldenummer: 09004922.2
(22) Anmeldetag: 02.04.2009
(51) Int. Cl.: A23L 1/305, A61K 38/16, A61P 19/00, A61P 1/00

(54) **Verwendung einer Zusammensetzung, welche ein Pflanzenlektin, ein proteolytisches Enzym und eine Selenverbindung umfasst, zum Schleimhautschutz und zur Linderung anderer Symptome bei Krebspatienten**
Use of a compound comprising a vegetable lectin, a proteolytic enzyme and a selenium compound for mucous membrane protection and relief of other symptoms experienced by cancer patients
Utilisation d'une composition, laquelle comprend une lectine végétale, un enzyme protéolytique et un composé de sélénium, destinée à la protection des muqueuses et à la réduction d'autres symptômes de patients atteints de cancer

(30) Priorität: 02.04.2008 EP 08006755
(43) Veröffentlichungstag der Anmeldung: 07.10.2009
(73) Patentinhaber: Helvista AG, 6300 Zug (CH)
(72) Erfinder:
(74) Vertreter: Helbig, Christian

(56) Entgegenhaltungen:
- EP-B- 0 942 741
- US-A1- 2007 031 098
- ANONYMOUS: "Equizym MCA Package Insert" INTERNET ARTICLE, [Online] Oktober 2007 (2007-10), XP002482311 Gefunden im Internet: URL:http://www.mycare.de/home_6640019_Equi zym%2BMCA__Suche_KYBERG%2BVERT.GMBH%2526CO .KG_0_.html> [gefunden am 2008-05-29]
- J. BREUTH: "Evidenzbasierte Komplementäronkologie" DER ONKOLOGE, Bd. 13, 14. April 2007 (2007-04-14), Seiten 534-542, XP019519712
- LEO AUERBACH: "Complementary and alternative medicine in the treatment of prostate cancer" JOURNAL OF MEN'S HEALTH AND GENDER, Bd. 3, Nr. 4, 31. Oktober 2006 (2006-10-31), Seiten 397-403, XP002482312 Elsevier Ireland Ltd.

## Beschreibung

### Hintergrund der Erfindung

Die vorliegende Erfindung betrifft Zusammensetzungen, die als Nahrungsergänzungsmittel, insbesondere für die bilanzierte Diät, oder als Arzneimittel geeignet sind, und deren Verwendung zum Schutz und/oder zur funktionellen Verbesserung und Unterstützung der Regeneration von Schleimhautgewebe sowie zur gleichzeitigen Linderung eines Spektrums anderer Symptome bei Krebspatienten, die sich einer onkologischen Strahlenoder Chemotherapie unterziehen oder unterzogen haben.

Nahrungsergänzungsmittel sind allgemein Lebensmittel, die üblicherweise in Form von Tabletten, Dragees, Kapseln oder als Pulver oder Granulat angeboten werden. Sie dienen dazu, die Nahrung mit bestimmten essenziellen (lebensnotwendigen) Mikronährstoffen (u.a. Vitaminen, Spurenelementen, Mineralstoffen, sekundären Pflanzenstoffen, prebiotischen Ballaststoffen) zu ergänzen.

Eine spezielle Form dieser Nahrungsergänzungsmittel sind Präparate für die bilanzierte Diät. Neben den apothekenpflichtigen Arzneimitteln, die meist verschreibungspflichtig sind und von den Krankenkassen erstattet werden, jedoch zum Teil auch als frei verkäufliche Präparate erhältlich sind, wurden ferner gesundheitsfördernde, die Ernährung optimierende, bilanzierte Diäten (BD) entwickelt. In Deutschland zugelassene und offiziell erhältliche Präparate für die bilanzierte Diät unterliegen einer behördlichen Qualitätsprüfung/kontrolle. Bilanziert bedeutet in diesem Zusammenhang, dass die Konzentrationen der enthaltenen Einzelbestandteile den täglichen Bedarf decken und somit die Ernährung optimieren. Die Begrenzung der Einzelkomponenten der Mikronährstoffe auf bestimmte Höchstkonzentrationen unterscheidet u.a. Präparate für die bilanzierte Diät von Arzneimitteln. Da die vorbeugende/therapeutische Wirkung von Vitaminen, Spurenelementen, sekundären Pflanzenstoffen usw. aber nicht ausschließlich von den Konzentrationen der Einzelmoleküle (Einzelbestandteile) sondern insbesondere auch von deren gegenseitiger Beeinflussung (Interaktion) abhängt, können Präparate für die bilanzierte Diät in bestimmten Lebens-/Krankheitsphasen sehr vorteilhaft sein.

Neben dem weitverbreiteten Wunsch, durch gezielte Vorsorge (u.a. durch Ernährungsoptimierung mittels ausgewogener Nahrungsaufnahme sowie durch Einnahme von Präparaten bilanzierter Diät; sportlicher Betätigung; psychosozialer Fürsorge) die Entstehung von Krankheiten bzw. deren Wiederauftreten (insbesondere von Krebs) zu verhindern, möchten Krebspatienten/Innen durch derartige Maßnahmen die Wirkung der (Krebs)Standardtherapien (Chemo-/Strahlentherapie) verbessern bzw. deren Nebenwirkungen (unerwünschte Arzneimittelwirkungen) reduzieren. Dies ist insofern wichtig und erwünscht, da die Reduktion von Nebenwirkungen der Standardtherapien deren optimale Anwendung bzgl. Dosierung und zeitlicher Abfolge ermöglicht und somit die Heilungschancen für Patienten/Innen verbessert.

Als wirksame Bestandteile solcher Präparate sind unter anderem hydrolytische Enzyme, insbesondere proteolytische Enzyme, Selen und pflanzliche Lektine bekannt.

Enzymhaltige Präparate werden z.B. zur unterstützenden Langzeitbehandlung von bösartigen Tumoren und zur unterstützenden Langzeitbehandlung von Entzündungen und Gefäßerkrankungen eingesetzt (vgl. z.B. WO 2004/100981). Diese Enzyme können sowohl pflanzlicher als auch tierischer Herkunft sein.

Bromelain (Rohextrakt aus Ananas) und Papain (Rohextrakt aus Papaya) sind Beispiele für pflanzliche, eiweißspaltende Enzyme (so genannte Cystein-Proteasen), deren naturheilkundliche Bedeutung seit Jahrtausenden bekannt ist. Obgleich die Wirksamkeit bestimmenden Bestandteile und Basismechanismen bislang nur teilweise erforscht wurden, sind entzündungshemmende, antiödematöse, antithrombotische/fibrinolytische Aktivitäten experimentell und klinisch dokumentiert. Pflanzliche Enzyme werden als Mono(Einzel)-Präparate (Bromelain, Papain) oder als Kombinationspräparate (z.T. in Kombination mit Enzymen tierischen Ursprungs wie Trypsin, Chymotrypsin) als orale Enzymtherapie unter anderem in der komplementären Krebsbehandlung verabreicht.

Die orale Enzymtherapie zeigte in wissenschaftlichen, experimentellen Untersuchungen immunstimulierende/immunmodulierende, antitumorale, antimetastatische und antiinfektiöse Aktivitäten. Auf dieser Grundlage wurden wissenschaftlich fundierte klinische Studien zur Unbedenklichkeit und Wirksamkeit der systemischen Enzymtherapie bei Krebspatienten/Innen komplementär zur Standardtherapie durchgeführt und ergaben u.a.
1. eine extrem niedrige Rate unerwünschter Arzneimittelwirkungen (= Unbedenklichkeit);
2. signifikant reduzierte Nebenwirkungen der Chemo/Strahlen-therapie (= Wirksamkeit);
3. signifikant verbesserte Lebensqualität unter Chemo-/Strahlentherapie (= Wirksamkeit);
4. von Tumorart und Tumorstadium abhängige Verlängerungen krankheitsfreier Intervalle (= Wirksamkeit).

Selen ist ein essenzielles Spurenelement, das dem Körper zugeführt werden muss. Mit der Nahrung wird biologisch/organisch gebundenes Selen aus Pflanzen (überwiegend als Selenomethionin) und aus tierischen Nahrungsmitteln (überwiegend als Selenocystein) aufgenommen. Zu den selenreichen Lebensmitteln zählen Fisch, Fleisch, Vollkorn, Hülsenfrüchte sowie Nüsse.

Anorganisch gebundenes Selen (überwiegend als Na-Selenit) wird in hohen Dosen als Arzneimittel betrachtet, ist jedoch in niedrigen Dosen Bestandteil von Nahrungsergänzungsmitteln, z.B. Multivitaminpräparaten. Es ist in Form von Tabletten, Trinkampullen und Injektionslösungen in Apotheken erhältlich. Na-Selenit ist physiologisch von Vorteil, da es für den Organismus direkt verfügbar ist und nicht, wie biologisch/organisch an Hefe oder Aminosäuren gebundenes Selen, in der Leber von der Trägersubstanz abgespalten werden muss.

Selen hat im Stoffwechsel des menschlichen Körpers vielfältige Aufgaben und ist für die Aufrechterhaltung von Stoffwechsel- und Organfunktionen notwendig. Diverse Selenoproteine/enzyme, z.B. die Glutathionperoxidase als wesentliche Komponente des antioxidativen Schutzsystems, entfalten Aktivität nur bei Verfügbarkeit von Selen.

Die komplementäre Gabe von Selen in Form von z.B. Na-Selenit während einer Chemo-/Strahlentherapie hat sich in wissenschaftlich fundierten Untersuchungen und Studien als unbedenklich und wirksam erwiesen, da u.a.
1. die Zytostatika-/Strahlenwirkung verstärkt wird;
2. Zytostatikaresistenzen reduziert werden;
3. durch die Therapie bedingte Nebenwirkungen von Chemo-/- Strahlentherapien, z.B. Herz-/Nierentoxizität, Lymphödem-/Erysipelentwicklung, reduziert werden;
4. Immunkompetenz und Allgemeinbefinden (Lebensqualität) verbessert werden.

Lektine sind zuckerbindende Eiweiße mit hoher Spezifität für definierte Zucker. Sie sind in der Natur weit verbreitet und haben große Bedeutung
1. in der Erkennung, Anlagerung und Haftung von Mikroorganismen (Bakterien, Viren, Parasiten) und Zellen (u.a. Organzellen, Tumorzellen);
2. als konzentrationsabhängige Zytotoxine (Zellgifte) bzw. Immunmodulatoren (u.a. in Pflanzen, z.B. Mistel, Hülsenfrüchte).

Die dosisabhängigen zytotoxischen und immunmodulierenden Eigenschaften definierter Mistellektine sind hinreichend dokumentiert und haben zur Akzeptanz der standardisierten Mistelextrakttherapie (von Tumorart und Tumorstadium abhängig) beigetragen. Die Indikationen der Mistelextrakttherapie umfassen derzeit
1. Immunsuppression nach Chemo-/Strahlentherapie (verzögerte Normalisierung von Immunzellzahlen und -aktivitäten im Blut);
2. Tumorart spezifisch (z.Z. studienmäßig belegt für Brustkrebs) Reduktion von Nebenwirkungen der Chemo-/Strahlentherapie.

Mistellektine können nicht oral verabreicht werden, da sie sehr toxisch sind und die Zellen im Magen-Darm-Trakt zerstören würden. Daher erfolgt die Gabe von Mistellektinen immer durch eine systemische (i.v., s.c., i.c.) Injektion.

Die immunmodulierenden Eigenschaften von pflanzlichen Lektinen beruhen nach bisherigen Erkenntnissen weitgehend auf der Aktivierung von Abwehrzellen durch Bindung an spezifische Zucker der Zellmembran.

Viele Patienten leiden während und/oder nach einer Strahlenoder Chemotherapie unter einem Komplex von Symptomen, der speziell die Schleimhäute und Gelenke betrifft. Solche Symptome sind beispielsweise trockene Schleimhäute und Schmerzen. Diese Symptome wurden bisher in herkömmlicher Weise durch z.B. Verabreichung bekannter schleimhautregenerierender Mittel oder Inhalationen/Spülungen mit Salzlösungen bzw. Schmerzmittel behandelt. Weitere typische Symptome solcher Patienten sind Übelkeit, Erbrechen/Durchfall, Müdigkeit/- Schwäche und Hautreizung, die gewöhnlich mit herkömmlichen Medikamenten für diese Indikationen behandelt werden. Diese herkömmlichen Behandlungen bringen jedoch in der Regel nur einen partiellen Erfolg und/oder sind mit Nebenwirkungen verbunden.

Die Druckschrift EP 0 942 741 B1 offenbart eine Zusammensetzung, welche als wirksamen Bestandteil ein Pflanzenlektin oder eine Mischung von Pflanzenlektinen umfasst, zur Verringerung und/oder zur Behandlung eines Schadens von Schleimhautzellen und/oder -geweben, insbesondere im Darm.

Vor diesem Hintergrund bestand eine Aufgabe der vorliegenden Erfindung in der Bereitstellung verbesserter schleimhautschützender Mittel, welche begleitend zu einer Chemo-/Strahlentherapie eingesetzt werden können und auf effektive und nebenwirkungsfreie Weise für den Schutz und/oder die Regeneration des angegriffenen bzw. gefährdeten Schleimhautgewebes sorgen. Eine speziellere Aufgabe war die Bereitstellung verbesserter schleimhautschützender Mittel, welche gleichzeitig auch bestimmte andere Symptome bei Krebspatienten lindern können.

Es wurde nun in klinischen Beobachtungen überraschenderweise festgestellt, dass die schleimhautschützende Wirkung bestimmter Pflanzenlektine, insbesondere Lens culinaris Lektin oder andere Lektine aus Hülsenfrüchten, durch die gleichzeitige Verabreichung von proteolytischen Enzymen und Selen in synergistischer Weise verstärkt wird. Darüber hinaus wurde festgestellt, dass diese Kombination auch eine Reihe anderer Symptome, insbesondere Erbrechen/Durchfall. Müdigkeit/Schwäche, Hautreizung und Antriebsschwache, bei Krebspatienten in synergistischer Weise zu lindern vermag. Eine derartig- Kombination von Wirkstoffen wird in der oben genannten Druckschrift EP 0 942 741 B1 weder Offenbart noch vorgeschlagen.

Somit werden die obigen Aufgaben erfindungsgemäß gelöst durch die Verwendung einer Zusammensetzung, welche mindestens ein Pflanzenlektin, mindestens ein proteolytisches Enzym und mindestens eine Selenverbindung umfasst, zur Herstellung eines Arzneimittels oder Nahrungsergänzungsmittels zur oralen Verabreichung, insbesondere für die bilanzierte Diät, zur gleichzeitigen Linderung eines Spektrums von Symptomen, welche Übelkeit, Erbrechen/Durchfall, trockene Schleimhäute, Gelenkbeschwerden, Müdigkeit/Schwäche und Hautreizung umfassen, bei Individuen, die sich einer onkologischen Strahlenund/oder Chemotherapie unterziehen oder unterzogen haben, wobei das Arzneimittel oder Nahrungsergänzungsmittel in einer solchen Menge verabreicht wird, dass eine Tagesdosis von mindestens 15-20 mg Lektin, 300-400 mg oder 1500-2000 FIP-Einheiten Papain, 300-400 mg oder 1500-2000 FIP-Einheiten Bromelain und 50-300 µg Natriumselenit erreicht wird.

Weiterhin werden die oben genannten Aufgaben gelöst duch eine Verwendung von Zusammensetzungen mit den oben genannten drei Komponenten, wie in den vorliegenden Ansprüchen 2 bis 7 näher definiert, zur Herstellung von Nahrungserganzungsmitteln oder Arzneimitteln mit hoher schleimhautschützender Wirkung und gleichzeitiger Wirkung zur Linderung bestimmter anderer Symptome bei Krebspatienten.

Die Erfindung stellt somit Nahrungsergänzungsmittel bzw. Arzneimittel bereit, die vorteilhaft verwendet werden können bei Patienten, die sich einer onkologischen Strahlen- und/oder Chemotherapie unterziehen oder unterzogen haben. Der Erfolg der erfindungemäßen Behandlung liegt unter anderem darin, dass primäre und sekundäre negative Begleiterscheinungen der Behandlung gelindert werden konnten. In vorteilhafter Weise erzielt die erfindungsgemäße Verwendung der beschriebenen Nahrungsergänzungsmittel bzw. Arzneimittel eine erhebliche Verbesserung des Gesamtstatus der Patienten. Dies wirkt sich unmittelbar positiv auf die Patienten aus und führt zu einer Verbesserung der Lebensqualität für die Patienten. Auch dürfen die positiven Wirkungen auf die primäre onkologische Behandlung nicht unterschätzt werden. Mit Hilfe der erfindungsgemäß verwendeten Nahrungsergänzungsmittel bzw. Arzneimittel wird die Primärbehandlung vielfach erst einigermaßen erträglich. Dies führt zu einer positiveren Einstellung der Patienten gegenüber der Behandlung und hat einen direkten, positiven Einfluss auf den Krankheitsverlauf und den Behandlungserfolg. Weiterhin vermögen die erfindungsgemäßen Nahrungsergänzungsmittel bzw. Arzneimittel im Nachgang zu einer derartig gravierenden onkologischen Behandlung als begleitende Maßnahme die weitere Genesung des Patienten positiv zu unterstützen.

Der Begriff "Schleimhautschutz", wie hier verwendet, umfasst allgemein den Schutz und/oder die Regeneration von angegriffenem bzw. gefährdetem Schleimhautgewebe und/oder die Optimierung der physiologischen Schleimhautfunktion, insbesondere auch in den Gelenken.

Als Lektine zur Verwendung in einem erfindungsgemäßen schleimhautschützenden Präparat eignen sich grundsätzliche alle Pflanzenlektine, welche die oben festgestellten synergistischen Wirkungen in Kombination mit proteolytischen Enzymen und einer Selenverbindung aufweisen. Spezielle, nicht beschränkende Beispiele hierfür sind u. a. Mistellektine: *Viscum album* Agglutinine (*Viscum album*), Lektine aus Hülsenfruchtpflanzen, vorzugsweise Erbse: *Pisum sativum* Agglutinin (*Pisum sativum*) und Linse: *Lens culinaris* Lektin (*Lens culinaris*), Erdnuss: *Arachis hypogaea* (Peanut Agglutinin), Getreide (Wheat Germ Agglutinin), Jackbohne: *Canavalia ensiformis* (Concanavalin A), Brennessel: *Urtica major* (Urtica Agglutinin) einschließlich synthetisch oder gentechnisch hergestellter Produkte oder Derivate davon.

Das gemäß Anspruch 1 Lektin oder die Lektine wird/werden in ausreichender Menge verabreicht, damit die gewünschten Wirkungen, insbesondere schleimhautschützende Wirkung und Linderung weiterer Symptome wie Erbrechen/Durchfall, Müdigkeit/Schwäche, Hautreizung, Antriebsschwache etc., bei Krebapetienten erzielt wird. Die Lektine werden in einer Tagesdosis von vorzugsweise 15-25 mg verabreicht .

Die verwendete Menge kann in Abhängigkeit von den speziellen Bedürfnissen des Konsumenten/Patienten variieren und unter Umständen auch außerhalb der genannten Bereiche liegen.

Vorzugsweise enthält die erfindungsgemäß verwendete Zusammensetzung die Lektine in einem natürlichen Extrakt, z.B. standardisierter Extrakt aus Linsen, Erbsen oder anderen Hülsenfrüchten.

Als proteolytische Enzyme für die erfindungsgemäße Verwendung kommen grundsätzlich alle proteolytischen Enzyme in Betracht, die in Kombination mit Lektinen und Selen eine synergistische Wirkung, insbesondere synergistische schleimhautschützende Wirkung und Wirkung bei Linderung weiterer Symptome wie Erbrechen/Durchfall, Müdigkeit/Schwäche, Hautreizung, Antriebsschwäche etc., bei Krebspatienten aufweisen. Diese Enzyme können sowohl tierischer als auch pflanzlicher Herkunft sein.

Einige nicht-beschränkende Beispiele für proteolytische Enzyme tierischer Herkunft sind Trypsin und Chymotrypsin sowie Pepsin. Diese Enzyme und oder aktive Derivate davon können auch synthetisch und/oder mit Hilfe gentechnisch manipulierter Mikroorganismen hergestellt werden.

Bevorzugte, jedoch nicht-beschränkende Beispiele für proteolytische Enzyme pflanzlicher Herkunft sind Ficin (aus (Feigen), Bromelain (aus Ananas) und Papain (aus Papaya). Bromelain besteht aus mehreren proteolytisch wirkenden Enzymen, spezielle Endopeptidasen, die aus den Stielen der Fruchtstände reifer oder unreifer Ananas gewonnen werden können. Papain ist eine Proteinase, die aus dem Milchsaft unreifer Papayafrüchte gewonnen werden kann. Die Enzyme können in reiner Form oder in Form von Pflanzenextrakten (z.B. Ananasextrakten, Papayaextrakten) verwendet werden. Gewünschtenfalls können auch synthetisch oder gentechnisch hergestellte Produkte oder Derivate der natürlichen Enzyme verwendet werden. Die Enzyme können sowohl einzeln als auch in Kombination verwendet werden. Eine bevorzugte Enzymkombination umfasst oder besteht aus Bromelain und Papain.

Die Enzyme werden in ausreichender Menge verabreicht, damit die gewünschten Wirkungen erzielt werden. Typischerweise werden die Enzyme in einer Tagesdosis von vorzugsweise 300-500 mg, pro Enzym verabreicht. In FIP-Einheiten ausgedrückt, werden die Enzyme typischerweise in einer Tagesdosis von vorzugsweise 1500-2500, FIP-Einheiten verabreicht. 1 FIP-Einheit entspricht der Enzymmenge, die in 1 min unter Standarbedingungen 1 µmol Substrat umsetzt. Nachdem die spezifische Aktivität von Enzympräparationen schwanken kann, ermöglicht die Angabe in FIP-Einheiten einen besseren Vergleich verschiedener Präparationen. Bei den in der vorliegenden Anmeldung verwendeten Enzymepräparaten entsprechen 100 mg Enzym 500 FIP-Einheiten. Die verwendete Menge kann in Abhängigkeit von den speziellen Bedürfnissen des Konsumenten/Patienten variieren und unter Umständen auch außerhalb der genannten Bereiche liegen.

Nachdem festgestellt wurde, dass die Wirkung von Lektinen und proteolytischen Enzymen durch die Anwesenheit von Selen synergistisch verstärkt wird, enthält die erfindungsgemäß verwendete Zusammensetzung auch Selen in einer physiologisch akzeptablen und vom Körper verwertbaren Form. Typischerweise handelt es sich dabei um eine anorganische Selenverbindung, vorzugsweise um Ammoniumselenit, ein Alkaliselenit, z.B. Natrium- oder Kaliumselenit, ein Erdalkaliselenit, z.B. Magnesiumselenit, oder um eine physiologisch äquivalente Verbindung. Diese Verbindungen, insbesondere Natrium- oder Kaliumselenit, werden sehr gut resorbiert und entfalten eine hohe biologische Aktivität.

Die Selenverbindung wird in ausreichender Menge verabreicht, dass die gewünschten Wirkungen, insbesondere schleimhautschützende Wirkung und Wirkung bei Linderung weiterer Symptome wie Erbrechen/Durchfall, Müdigkeit/Schwäche, Hautreizung, Antriebsschwäche etc., bei Krebspatienten erzielt werden. Typischerweise wird das Selen in einer Tagesdosis, die vorzugeweise 50 µg bis 400 µg, vorzugsweise 75-300 µg, z.B. 75-200 µg oder 225-300 pg, Natriumselenit oder einer äquimolaren Menge einer anderen Selenverbindung entspricht, verabreicht werden. Die verwendete Menge kann in Abhängigkeit von den speziellen Bedürfnissen des Konsutnenten/Patienten variieren und unter Umständen auch außerhalb der genannten Bereiche liegen.

Eine besonders bevorzugte und wirksame Kombination zur erfindungsgemäßen Verwendung umfasst 15-20 mg Lektin aus Lens culinaris, 300-400 mg oder 1500-2000 FIP-Einheiten Bromelain, 300-400 mg oder 1500-2000 FIP-Einheiten Papain und 225-300 µg Natriumselenit. Ein besonderer Vorteil der erfindungsgemäß bevorzugten Lektine aus Hülsenfrüchten besteht darin, dass sie im Gegensatz zu Mistellektinen oral verabreicht werden können und ihre schleimhautschützende Wirkung und anderen Wirkungen auch in oraler Form entfalten.

Beispiele für die orale Verabreichung sind Tabletten, Dragees, Kapseln, Pulver oder Granulat. Besonders bevorzugt liegt die Zusammensetzung in Form einer Tablette oder Filmtablette vor. Insbesondere für den Fall, dass eine zeitliche gesteuerte oder verzögerte Freisetzung einzelner oder aller Komponenten gewünscht wird, kann auch eine Kapsel eine bevorzugte Darreichungsform sein.

Die erfindungsgemäß verwendeten schleimhautschützenden Komponenten können auch in Mischung mit anderen physiologisch wirksamen Bestandteilen vorliegen.

Typischerweise werden die physiologisch wirksamen Komponenten in Mischung mit herkömmlichen und physiologisch verträglichen Trägern, Füll- und Bindemitteln, Einschlussmitteln, z.B. mit einer magensaftresistenten Hülle, optional auch mit Geschmacksstoffen, vorliegen. Solche Zusatzstoffe können beispielsweise aus Reisstärke, Maistärke, Stearinsäure, Magnesiumstearat, mikrokristalliner Cellulose, Hydroxypropylcellulose, Hydroxymethylcellulose, Talkum, Siliumdioxid, Magesiumoxid, Titanoxid, Silikaten, Gelatine, Crosspovidon, Schellack und anderen üblichen Zusätzen ausgewählt sein. Die Herstellung und Mischung geeigneter Zusammensetzungen und Darreichungsformen kann nach den üblichen Verfahren des Standes der Technik zur Herstellung von pharmazeutischen Produkten und Nahrungsergänzungsmitteln erfolgen.

Die Einnahme der erfindungsgemäß verwendeten schleimhautschützenden Präparate kann einmal oder mehrmals täglich erfolgen. Jeweils eine Stunde vor und eine Stunde nach der Einnahme von enzymhaltigen bilanzierten Diäten sollte keine Nahrungsaufnahme erfolgen. Bei der Darreichungsform als Tabletten hat sich die Einnahme von 1-2 Tabletten morgens und a-bends 1 Stunde vor einer Hauptmahlzeit als effektiv und gut verträglich erwiesen. Noch günstiger ist eine Verabreichung von einer Tablette 4 X täglich.

Die erfindungsgemäß verwendeten natriumselenithaltigen Präparate sollten nicht zusammen mit Vitamin C-haltigen Präparaten, Speisen oder Getränken eingenommen werden, Na-Selenit wird in Verbindung mit Vitamin C in eine für den Organismus nicht verwertbare Form umgewandelt. Daher sollte zwischen der Aufnahme dieser Präparate und Vitamin C mindestens 1 Stunde Abstand eingehalten werden.

### Figurenbeschreibung

Fig. 1 stellt den Einfluss der Verabreichung einer erfindungsgemäß verwendeten Zusammensetzung auf die Verträglichkeit einer Chemo-/Strahlentherapie bei Mammakarzinompatientinnen graphisch dar.
Fig. 2 stellt den Einfluss der gleichen Zusammensetzung auf verschiedene Symptome, insbesondere trockene Schleimhäute, als Folge der Chemo-/Strahlentherapie graphisch dar.

Die folgenden Anwendungsbeispiele sollen die vorliegende Erfindung näher erläutern, jedoch keineswegs darauf beschränken.

### BEISPIEL 1

Eine Zusammensetzung, die *Lens culinaris* Lektin, Natriumselenit sowie Papain und Bromelain umfasste, (Herstellerbezeichnung Equizym) wurde bei Mammakarzinompatientinnen, die sich einer adjuvanten Chemo- (FEC oder TAC) und Strahlentherapie unterzogen, getestet. Von 25 Patientinnen erhielten 13 diese Zusammensetzung und die restlichen 12 bildeten die Vergleichsgruppe. Die Zusammensetzung wurde in Tablettenform (4 Tabletten pro Tag) von Beginn der Chemo- bis Ende der Strahlentherapie verabreicht. Die übliche Tagesdosis betrug 20 mg Lektin, 400 mg (2000 FIP-E.) Papain, 400 mg (2000 FIP-E.) Bromelain und 300 µg Natriumselenit.

Die Wirkung wurde von den Patientinnen nach einem vorher festgelegten Score (1 = keine Symptome bis 6 = stärkste Symptome) selbst bewertet.

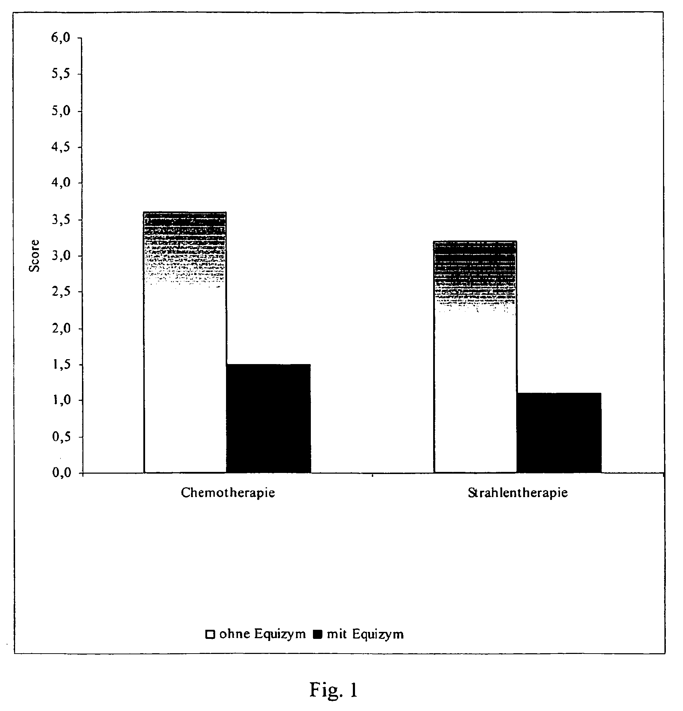

Der Einfluss der Zusammensetzung auf die Gesamtverträglichkeit der Chemo-/Strahlentherapie ist in Tab. 1 zusammengefasst und in Fig. 1 graphisch dargestellt.

**Tabelle 1**

| Verträglichkeit | Ohne Equizym (n = 12) | Mit Equizym (n = 13) |
|---|---|---|
| Chemotherapie | 3,6 | 1,5 |
| Strahlentherapie | 3,2 | 1,1 |

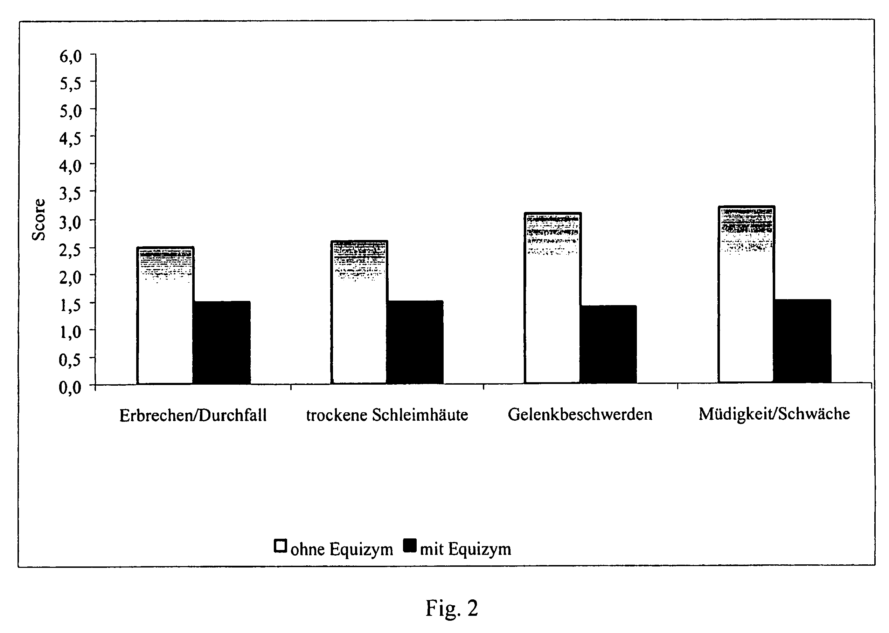

Der Einfluss der Zusammensetzung auf verschiedene Symptome, insbesondere trockene Schleimhäute und damit in Zusammenhang stehende Gelenkbeschwerden, als Folge der Chemo-/Strahlentherapie ist in Tab. 2 zusammengefasst und in Fig. 2 graphisch dargestellt.

**Tabelle 2**

| Symptome der Chemo-/Strahlentherapie | Ohne Equizym (n = 12) | Mit Equizym (n = 13) |
|---|---|---|
| Übelkeit | 2,9 | 1,7 |
| Erbrechen/Durchfall | 2,5 | 1,5 |
| Trockene Schleimhäute | 2,6 | 1,5 |
| Gelenkbeschwerden | 3,1 | 1,4 |
| Müdigkeit/Schwäche | 3,3 | 1,5 |
| Hautreizung | 2,5 | 1,3 |

Aus diesen Daten ist ersichtlich, dass die Verabreichung der Zusammensetzung zu einer erheblich gesteigerten Verträglichkeit der Chemo-/Strahlentherapie insgesamt und zu einer erheblichen Linderung verschiedener Symptome, insbesondere des Symptoms der trockenen Schleimhäute und der Gelenkbeschwerden, führt.

### BEISPIEL 2

Zur Untersuchung des Einflusses einzelner Komponenten oder paarweiser Kombinationen der Einzelkomponenten wurden weitere Tests durchgeführt. Die grundsätzliche Vorgehensweise bei der Verabreichung und der Beurteilung der Wirkung durch die Patienten war wie in Beispiel 1 beschrieben. Die jeweils verabreichten Mengen der verschiedenen Präparate und die beobachteten Wirkungen sind in den folgenden Tabellen zusammengefasst. Die Verabreichungsbedingungen für Tabelle 3 entsprechen denen von Tabelle 2 aus Beispiel 1, diese Untersuchung wurde jedoch mit einer größeren Anzahl von Probanden durchgeführt.

**Tabelle 3**

| Symptome der Chemo-/Strahlentherapie | Ohne Equizym (n = 24) | Mit Equizym 4x1 Tabletten/die (n = 26) |
|---|---|---|
| Übelkeit | 2,9 | 1,7 |
| Erbrechen/Durchfall | 2,5 | 1,5 |
| trockene Schleimhäute | 2,6 | 1,5 |
| Gelenkbeschwerden | 3,1 | 1,4 |
| Müdigkeit/Schwäche | 3,3 | 1,5 |
| Hautreizung | 2,5 | 1,3 |
| Fatique, Antriebsschwäche | 3,8 | 1,2 |

**Tabelle 4**

| Symptome der Chemo-/Strahlentherapie | Nur Enzyme 4x täglich 100mg Bromelain, 100mg Papain (n = 14) | Nur Na-Selenit 4x täglich 75µg NaSe (n = 14) |
|---|---|---|
| Übelkeit | 2,6 | 2,3 |
| Erbrechen/Durchfall | 3,0 | 2,1 |
| trockene Schleimhäute | 2,6 | 2,6 |
| Gelenkbeschwerden | 2,5 | 2,8 |
| Müdigkeit/Schwäche | 3,1 | 2,7 |
| Hautreizung | 2,3 | 2,5 |
| Fatique, Antriebsschwäche | 3,8 | 3,6 |

**Tabelle 5**

| Symptome der Chemo-/Strahlentherapie | Nur Lektine 4x täglich 5mg Lektin (n = 10) | Nur Na-Selenit 4x täglich 75µg NaSe (n = 14) |
|---|---|---|
| Übelkeit | 2,7 | 2,3 |
| Erbrechen/Durchfall | 2,7 | 2,1 |
| Trockene Schleimhäute | 2,0 | 2,6 |
| Gelenkbeschwerden | 2,1 | 2,8 |
| Müdigkeit/Schwäche | 2,8 | 2,7 |
| Hautreizung | 2,2 | 2,5 |
| Fatique, Antriebsschwäche | 3,2 | 3,6 |

**Tabelle 6**

| Symptome der Chemo-/Strahlentherapie | Enzyme plus Na-Selenit: 4x täglich 100mg Bromelain, 100mg Papain, 75µg NaSe (n = 14) | Enzyme plus Lektine: 4x täglich 100mg Bromelain, 100mg Papain, 5mg Lektin (n = 18) |
|---|---|---|
| Übelkeit | 2,6 | 2,4 |
| Erbrechen/Durchfall | 2,6 | 2,7 |
| Trockene Schleimhäute | 2,6 | 2,0 |
| Gelenkbeschwerden | 2,4 | 1,9 |
| Müdigkeit/Schwäche | 2,8 | 2,5 |
| Hautreizung | 2,1 | 2,0 |
| Fatique, Antriebsschwäche | 3,4 | 3,2 |

Diese Daten belegen einen sehr ausgeprägten synergistischen Effekt der Dreierkombination aus Lektinen, proteolytischen Enzymen und Selenverbindung bei der Behandlung des Symptoms der trockenen Schleimhäute. Die Tabellen 3 und 4 zeigen, dass die Verabreichung von Enzymen oder Selenverbindung allein praktisch keine Wirkung hat (der Wert von 2,6 ohne Behandlung bleibt unverändert), während Tabelle 6 zeigt, das dies auch für die Verabreichung einer Kombination von Enzymen und Selen gilt (auch hier resultiert nur ein Wert von 2,6). Demgegenüber zeigen die Tabellen 5 und 6, dass die Verabreichung von Lektinen allein eine deutliche Wirkung hat (Verbesserung des Wertes auf 2,0), welche durch eine Kombination von Lektinen mit Enzymen nicht mehr verbessert wird. Erst die Verabreichung der Dreierkombination führt zu einer nochmaligen deutlichen Verbesserung des Werts auf 1,5, wie die Tabellen 2 und 3 zeigen.

Auch für die Behandlung der Symptome Erbrechen/Durchfall, Müdigkeit/Schwäche, Hautreizung und Antriebsschwäche ist ein deutlicher synergistischer Effekt zu beobachten. Bei den anderen Symptomen ist die Synergie zwar vorhanden, jedoch weniger offensichtlich.

Die Tabelle 4 zeigt, dass die Verabreichung von Enzymen allein dass Symptom Erbrechen/Durchfall sogar verschlimmert (der Wert von 2,5 ohne Behandlung steigt auf 3,0), während die Tabellen 5 und 6 zeigen, dass die Verabreichung von Lektinen allein oder einer Kombination von Enzymen und Lektinen keine Wirkung oder eine geringfügig beeinträchtigende Wirkung hat (Wert von 2,7). Demgegenüber zeigen die Tabellen 4 und 5, dass die Verabreichung von Selen allein eine deutliche Wirkung hat (Verbesserung des Wertes auf 2,1), welche durch eine Kombination mit Enzymen verschlechtert wird (Wert von 2,6). Erst die Verabreichung der Dreierkombination führt zu einer drastischen Verbesserung des Werts auf 1,5, wie die Tabellen 2 und 3 zeigen.

Die Tabellen 3 bis 5 zeigen, dass das Symptom von Müdigkeit/Schwäche durch die Verabreichung von Enzymen, Lektinen oder Selenverbindung allein jeweils geringfügig gelindert wird (Verbesserung des Werts von 3,3 auf 3,1 bis 2,7), während die Verabreichung der Dreierkombination zu einer sehr starken Verringerung des Werts auf 1,5 führt.

Die Tabellen 3 bis 5 zeigen auch, dass das Symptom der Hautreizung durch die Verabreichung von Enzymen, Lektinen oder Selenverbindung allein nicht oder nur geringfügig gelindert wird (Verbesserung des Werts von 2,5 auf 2,3 bis 2,2), während die Verabreichung der Dreierkombination zu einer sehr starken Verringerung des Werts auf 1,3 führt.

In ähnlicher Weise zeigen die Tabellen 3 bis 5, dass das Symptom der Antriebschwäche durch die Verabreichung von Selen oder Lektinen allein jeweils geringfügig gelindert wird (Verbesserung des Werts von 3,8 auf 3,6 bzw. 3,2) und durch die Verabreichung von Enzymen allein gar nicht, während die Verabreichung der Dreierkombination zu einer sehr starken Verringerung des Werts auf 1,2 führt. Das Symptom der Antriebsschwäche unterscheidet sich von dem zuerst untersuchten Symptom der Müdigkeit/Schwäche darin, das es sich in erster Linie nicht um ein körperliches Symptom, sondern um ein mentales Problem handelt, das weitgehend unabhängig von der allgemeinen körperlichen Verfassung auftreten kann.

## Patentansprüche

1. Verwendung einer Zusammensetzung, welche mindestens ein Pflanzenlektin, mindestens ein proteolytisches Enzym und mindestens eine Selenverbindung umfasst, zur Herstellung eines Arzneimittels oder Nahrungsergänzungsmittels zur oralen Verabreichung, insbesondere für die bilanzierte Diät, zur gleichzeitigen Linderung eines Spektrums von Symptomen, welche Übelkeit, Erbrechen/Durchfall, trockene Schleimhäute, Gelenkbeschwerden, Müdigkeit/Schwäche und Hautreizung umfassen, bei Individuen, die sich einer onkologischen Strahlenund/oder Chemotherapie unterziehen oder unterzogen haben, wobei das Arzneimittel oder Nahrungsergänzungsmittel in einer solchen Menge verabreicht wird, dass eine Tagesdosis von mindestens 15-20 mg Lektin, 300-400 mg oder 1500-2000 FIP-Einheiten Papain, 300-400 mg oder 1500-2000 FIP-Einheiten Bromelain und 50-300 µg Natriumselenit erreicht wird.

2. Verwendung nach Anspruch 1, wobei die Zusammensetzung ein Lektin aus einer Hülsenfrucht, z.B. *Lens culinaris* Lektin, umfasst.

3. Verwendung nach Anspruch 1 oder 2, wobei die Selenverbindung aus einem Alkaliselenit wie Natriumselenit oder Kaliumselenit, einem Erdalkaliselenit wie Magnesiumselenit, Ammoniumselenit und anderen physiologisch verträglichen und vom Körper resorbierbaren Selenverbindungen ausgewählt ist.

4. Verwendung nach einem der Ansprüche 1-3, wobei die Zusammensetzung ein proteolytisches Enzym oder eine Mischung proteolytischer Enzyme, ausgewählt aus Bromelain, Papain, Trypsin und Chymotrypsin, umfasst.

5. Verwendung nach Anspruch 4, wobei die Zusammensetzung Bromelain und Papain umfasst.

6. Verwendung nach einem der Ansprüche 1-5, wobei die Zusammensetzung *Lens culinaris* Lektin, Natrium- oder Kaliumselenit sowie Bromelain und Papain umfasst.

7. Verwendung nach Anspruch 6, wobei dass Arzneimittel oder Nahrungsergänzungsmittel zur Verabreichung in einer solchen Menge bestimmt ist, dass eine Tagesdosis von mindestens 15-25 mg Lektin, 300-500 mg oder 1500-2500 FIP-Einheiten Papain, 300-500 mg oder 1500-2500 FTP-Einheiten Bromelain und 225-300 µg Natriumselenit erreicht wird.

## Claims

1. Use of a composition comprising at least one plant lectin, at least one proteolytic enzyme and at least one selenium compound, for the preparation of a medicament or a dietary supplement composition for oral application, in particular for the balanced diet, for the simultaneous amelioration of a range of symptoms, which symptoms comprise nausea, vomiting/diarrhea, dry mucous membranes, joint touble, fatigue/weakness and skin irritation, in subjects being treated or having been treated with a oncology irradiation and/or chemotherapy, wherein the medicament or the dietary supplement composition is administered in such a dosage that a daily dosis is achieved of at least 15-20 mg lectin, 300-400 mg or 1500-2000 FIP-units of papain, 300-400 mg or 1500-2000 FIP-units bromelin und 50-300 µg sodium selenite.

2. Use according to claim 1 wherein the composition comprises a lectin originating from peas and beans, e.g. lens culinaris lectin.

3. Use according to claim 1 or 2 wherein the selenium compound is selected from an alkali selenite, such as a sodium selenite or a potassium selenite, an earth alkali selenite, such as magnesium selenite, an ammonium selenite, and other physiologically acceptable and selenium compounds resorbable by the body.

4. Use according to any of claims 1 to 3 wherein the composition comprises a proteolytic enzyme or a mixture of proteolytic enzymes selected from a bromelin, a papain, a trypsin and a chymotrypsin.

5. Use according to claim 4 wherein the composition comprises bromelin and papain.

6. Use according to any of claims 1 - 5 wherein the composition comprises lens culinaris lectin, sodium or potassium selenite as well as bromelin and papain.

7. Use according to claim 6 wherein the medicament or the dietary supplement composition for application is meant to be used in such an amount that a daily dosis is achieved of at least 15-25 mg lectin, 300-500 mg or 1500-2500 FIP-units of papain, 300-500 mg or 1500-2500 FIP-units of bromelin und 225-300 µg sodium selenite.

## Revendications

1. Utilisation d'une composition, comprenant au moins une lectine végétale, au moins une enzyme protéolytique et au moins un composé de sélénium, pour la préparation d'un médicament ou d'un complément alimentaire destiné à être administré par voie orale, notamment pour la diète balancée, pour le soulagement simultané d'une variété de symptomes, dont la nausée, le vomissement/la diarrhée, les muqueuses sèches, les troubles articulaires, la fatigue/faiblesse et l'irritation cutanée, chez des individus qui se soumettent ou se sont soumis à une radiothérapie et/ou chimiothérapie oncologiques, ledit médicament ou complément alimentaire étant administré dans une quantité apte à atteindre une dose journalière d'au moins 15-20 mg de lectine, 300-400 mg ou 1500-2000 unités FIP de papaïne, 300-400 mg ou 1500-2000 unités FIP de bromélaïne, et 50-300 µg de sélénite de sodium.

2. Utilisation selon la revendication 1, dans laquelle ladite composition comprend une lectine obtenue à partir d'une légumineuse, par exemple une lectine de *Lens culinaris.*

3. Utilisation selon la revendication 1 ou 2, dans laquelle le composé de sélénium est choisi parmi un sélénite alcalin, tel que le sélénite de sodium ou de kalium, un sélénite alcalino-terreux, tel que le sélénite de magnésium, le sélénite d'ammonium, et d'autres composés de sélénium physiologiquement acceptables et résorbables par le corps.

4. Utilisation selon l'une quelconque des revendications 1 à 3, dans laquelle ladite composition comprend une enzyme protéoloytique ou une mixture d'enzymes protéoloytiques, choisie parmi la bromélaïne, la papaïne, la trypsine et la chymotrypsine.

5. Utilisation selon la revendication 4, dans laquelle ladite composition comprend de la bromélaïne et de la papaïne.

6. Utilisation selon l'une quelconque des revendications 1 à 5, dans laquelle ladite composition comprend de la lectine de *Lens culinaris,* du sélénite de sodium ou de kalium, ainsi que de la bromélaïne et de la papaïne.

7. Utilisation selon la revendication 6, dans laquelle ledit médicament ou complément alimentaire est destiné à être administré dans une quantité apte à atteindre une dose journalière d'au moins 15-25 mg de lectine, 300-500 mg ou 1500-2500 unités FIP de papaïne, 300-500 mg ou 1500-2500 unités FIP de bromélaïne, et 225-300 µg de sélénite de sodium.
